# EUROPÄISCHE PATENTSCHRIFT

(11) **EP 2 497 522 B1**
(45) Veröffentlichungstag und Bekanntmachung des Hinweises auf die Patenterteilung: **08.01.2014**
(21) Anmeldenummer: 12156179.9
(22) Anmeldetag: 20.02.2012
(51) Int. Cl.: A61M 25/06, B26D 3/00, B26D 7/02

(54) **Schlitzwerkzeug**
Slitter Tool
Outil de rainurage

(30) Priorität: 10.03.2011 DE 102011001191; 13.09.2011 US 201161533800 P
(43) Veröffentlichungstag der Anmeldung: 12.09.2012
(73) Patentinhaber: BIOTRONIK SE & Co. KG, 12359 Berlin (DE)
(72) Erfinder: Grauhan, Ole, 14163 Berlin (DE); Landgraf, Tassilo, 12249 Berlin (DE); Braun, Ramona, 13591 Berlin (DE)
(74) Vertreter: Lindner-Vogt, Karin L.

(56) Entgegenhaltungen:
- US-A- 5 330 460
- US-A1- 2005 182 435
- US-A1- 2009 054 840

## Beschreibung

Die Patentanmeldung beschreibt ein Schlitzwerkzeug zum Schneiden von Kathetern bei CRT-Implantationen, auch Slitter Tool genannt.

Bei der Implantation von Elektrodenleitungen werden Einführkatheter benutzt, in welcher sich die zu implantierende Elektrodenleitung befindet. Der Innendurchmesser des Einführkatheters ist dabei dem Außendurchmesser der Elektrodenleitung angepasst. Zuerst wird der Einführkatheter in das betreffende Gefäß geführt und anschließend durch den Einführkatheter die Elektrodenleitung in das Gefäß implantiert.

Die Elektrodenleitung weist dabei am proximalen Ende einen Elektrodenstecker auf, der zur elektrischen Verbindung mit einem elektromedizinischen Implantat genutzt wird. Dieser Stecker hat in der Regel einen Außendurchmesser, der größer ist als der Innendurchmesser des Einführkatheters. Da der Einführkatheter aber - wie der Name schon sagt - lediglich zum Einführen der Elektrodenleitung genutzt wird, muss er wieder entfernt werden. Ein einfaches Ziehen in proximaler Richtung ist dabei wegen des Elektrodensteckers nicht möglich.

Schlitzwerkzeuge werden zum Eröffnen des Einführkatheters benutzt, um diesen über den Elektrodenstecker zu entfernen, wenn er nicht mehr benötigt wird. Dabei wird der Einführkatheter mit Hilfe einer am Schlitzwerkzeug befindlichen Klinge der Länge nach aufgeschlitzt und dann entfernt. Problematisch bei dieser Prozedur ist, dass die soeben implantierte Elektrodenleitung auch verrutschen kann. Um dies zu vermeiden, sind vielfältige Klemmmechanismen bekannt. Ein Beispiel dafür ist der US 4,687,469 oder der US 7,338,481 zu entnehmen, bei der die Elektrodenleitung mit einem Finger des Operateurs am Schlitzwerkzeug manuell fixiert wird und der Einführkatheter darüber hinweggezogen wird. Nachteilig ist hierbei, dass die manuelle Fixierung sehr unzuverlässig ist und es versehentlich zu einem Verrutschen der schon implantierten Elektrodenleitung kommen kann. Weiterhin kann bei dieser Lösung die Elektrodenleitung durch Einknicken geschädigt werden. Zudem sind diese Lösungen meist nicht für unterschiedliche Elektrodenleitungsdurchmesser geeignet, weil die Klemmführung, in der mittels beispielsweise des Daumendruckes die Elektrodenleitung fixiert wird, nur für bestimmte Durchmesser dimensioniert ist. Gerade bei zu geringen Durchmessern kann dies zu einem Verrutschen führen.

Ebenso störanfällig ist auch der halbmanuelle Fixiermechanismus aus der US 6,497,681, der auf einer Klemmzange basiert, mittels der die Elektrodenleitung fixiert wird. Dazu muss der Operateur die Klemmzange aktiv zusammenpressen, um so die Elektrodenleitung zwischen den Klemmzangenschenkeln zu fixieren. Auch hier kann es zu einer versehentlichen Öffnung kommen, jedoch ist das Problem der Knickung der Elektrodenleitung minimiert worden.

Ein weiterer Lösungsvorschlag ist aus der US-Patentanmeldung 2009/0049698 bekannt, bei der die Fixierung der Elektrodenleitung mittels eines Befestigungsarms am Gehäuse des Schlitzwerkzeuges erfolgt. Die Fixierung wird durch das Betätigen eines Druckknopfes bewirkt, so dass der Operateur während des Schlitzvorganges einen konstanten Fingerdruck auf diesen Druckknopf ausüben muss. Diese Lösung ist für den Operateur nicht praktikabel, da sehr anstrengend. Dies wiederum kann zu elektrodenschädigendem Fehlverhalten führen. Eine ähnliche Lösung wird in der US 2009/0054840 offenbart; und zwar ein Haltegriff für einen Katheter, in dem ein Schlitzwerkzeug und ein Haltemechanismus integriert sind.

Auch das US-Patent US 5,330,460 und die US-Anmeldung US 2005/0182435 A1 zeigen Schlitzwerkzeuge, die jedoch in der Handhabung für den Operateur ebenfalls recht unpraktikabel sind.

Vor dem Hintergrund der genannten Nachteile des Standes der Technik besteht ein Bedarf an einem Schlitzwerkzeug, welches eine zuverlässige, verrutschsichere Fixierung von Elektrodenleitungen mit unterschiedlichem Durchmesser bei gleichzeitig vereinfachter Handhabung und Vermeidung von Elektrodenleitungsschädigungen bietet.

Das Schlitzwerkzeug gemäß einer Ausführung des Gegenstandes der Patentanmeldung umfasst ein Gehäuse, eine am Gehäuse befestigte Klinge, einen am Gehäuse befindlichen und von außen betätigbaren Druckknopf sowie im Gehäuse eine Klemmzange mit zwei (Klemm-)Schenkeln. Die Schenkel der Klemmzange sind so geformt, dass sie sich von einer Basis, an dem beide Schenkel miteinander befestigt sind, in Richtung einer Peripherie, in der die Elektrodenleitung fixiert wird, voneinander so weg bewegen, so dass der Abstand zwischen beiden Schenkeln erweitert ist. Im Ruhezustand der Klemmzange bewirkt jeder Schenkel eine Kraft durch eine Vorspannung, die jeweils gegen den anderen Schenkel gerichtet ist und so eine Klemmung an der Peripherie bewirkt.

Die soeben beschriebene Klemmzange ist fest mit dem Gehäuse verbunden und das Spreizelement ist verschieblich gelagert. Hier wirken der Druckknopf, das Spreizelement und die Klemmzange derart miteinander, dass eine Betätigung des Druckknopfes den Ruhezustand der Klemmzange aufhebt und eine Öffnung der Fixierung in der Peripherie der Klemmzange bewirken, indem die beiden Schenkel durch das Spreizelement voneinander weg bewegt werden.

In der Ausführungsform wird die Peripherie der Klemmzange durch die offenen Enden der Klemmschenkel der Klemmzange gebildet. Diese sind in ihrer Form so, dass sie die Fixierung der Elektrodenleitung, welche sich innerhalb des Katheters befindet, effektiv und sicher ermöglichen. Bevorzugt bestehen diese offenen Enden der Klemmschenkel aus je einer runden oder prismaförmigen Halbschale zur Aufnahme der Elektrodenleitung, wodurch die Fixierung der Elektrodenleitung während des Aufschlitzens des Katheters bewirkt wird.

Damit der Katheter in seiner Längsachse aufgeschlitzt werden kann, muss der Katheter in Verhältnis zur Klinge so positioniert werden, dass sie sich in weitgehend radialer Richtung zum Katheterquerschnitt befindet. Deshalb bilden die beiden soeben genannten Halbschalen der Klemmzange im Ruhezustand einen umschlossenen zylinderförmigen Raum, der eine Längsachse aufweist, die so angeordnet ist, dass die in diesem Raum befindliche Elektrodenleitung und damit den Katheter so zur Klinge positioniert sind, dass die Klinge den Katheter in seiner Längsrichtung aufschlitzen kann.

Um die Schneidbewegung noch zuverlässiger zu gestalten, umfasst die Klinge eine Führung, welche parallel zur Längsachse der Halbschale verläuft und welche während des Schneidens oder Schlitzens das Ausweichen des Katheters von der Klinge weg verhindert. Dazu befindet sich diese Führung am offenen Ende der Klinge, also an der Seite, die vom Gehäuse wegragt. Die Klinge befindet sich zwischen Führung und Gehäuse. So kann der Katheter zwischen der Führung und dem Gehäuse eingelegt werden.

Eine zentrale Funktion des beschriebenen Schlitzwerkzeugs kommt dem Druckknopf zu, der im Ruhezustand eine erste, aus dem Gehäuse herausragende Position und eine zweite, sich nach der Betätigung im Gehäuse befindliche Position aufweist und damit bewirkt, dass die Fixierung geöffnet ist. Durch die Betätigung dieses Druckknopfes wird das Zusammenwirken mit dem Spreizelement und der Klemmzange hervorgerufen, welches letztendlich in der Auseinanderbewegung der beiden Schenkel mündet. Dadurch, dass im Gegensatz zu den Lösungen im Stand der Technik der Mechanismus und damit die Kraftaufbringung auf das System erst zur Lösung der Fixierung notwendig sind, wird die Handhabung vereinfacht.

Damit die Klemmzange wieder in den Ruhezustand zurückfällt und das System aus Druckknopf, Spreizelement und Klemmzange weiterhin betätigbar bleibt, sind ein oder mehrere Rücksetzelemente vorgesehen, welche eine Rückbewegung des Druckknopfes aus der zweiten in die erste Position bewirken. Die Rücksetzelemente können Federn, insbesondere Schraubenfedern, welche zwischen Gehäuse und Druckknopf wirken, sein. Da der Druckknopf mit dem auslösenden Element - also mit dem Spreizelement - verbunden ist, führt eine Rückführung des Druckknopfes in die erste Position zum Schließen der Fixierung.

Bei Betätigung des Druckknopfes wird eine gegenseitige Verschiebung der Klemmzange und des Spreizelements bewirkt. Insbesondere verschieben sich dabei das Spreizelement und die Basis der Klemmzange aufeinander zu. Durch die soeben beschriebenen Rücksetzelemente ist dieser Vorgang ohne Krafteinwirkung jederzeit reversibel, wodurch eine einfache Handhabung durch den Operateur ermöglicht wird.

In der Regel befindet sich das Spreizelement zwischen den beiden Schenkeln, damit es bei Betätigung des Druckknopfes auf die beiden Schenkel wirken kann und diese so nach außen drücken bzw. auslenken kann. Das Spreizelement kann dabei den Querschnitt eines Dreiecks einnehmen, wobei eine der Ecken in Richtung der Basis der Klemmzange weist. So wird die Betätigung erleichtert. Zwischen den beiden Schenkeln im Abschnitt, in dem der Abstand erweitert ist, befindet sich ein Spreizelement. Bei Betätigung des Druckknopfes bewegen sich die beiden Schenkel bezüglich des Spreizelements, welches so positioniert und dimensioniert ist, dass es die beiden Schenkel auseinander drückt. Dadurch wird die Klemmung aufgehoben.

In der Ausführung, in der das Spreizelement mit dem Druckknopf verbunden und damit verschiebbar ist, führt die Betätigung des Druckknopfes zu einer Verschiebung des Spreizelements in Richtung der im Gehäuse fest gelagerten Basis der Klemmzange, wodurch bewirkt wird, dass die beiden Schenkel auseinander gedrückt werden.

Gemäß einer weiteren Variante dieser Ausführung nimmt das Spreizelement bei Betätigung des Druckknopfes die Position zwischen den beiden Schenkeln ein. Beispielsweise befindet sich dann das Spreizelement in der ersten Position des Druckknopfes lateral des Spaltes zwischen den zwei Schenkeln der Klemmzange. Durch Betätigung des Druckknopfes und Bewegung desselben in die zweite Position bewegt sich das Spreizelement in den Raum zwischen die zwei Schenkel, wodurch die Schenkel voneinander weg bewegt werden und damit die Klemmung geöffnet wird.

Die Fixierung der Elektrodenleitung läuft wir folgt ab: Es wird eine Klemmung (Fixierung) mit einem variablen Durchmesser gewählt. Dies ist nur durch eine aktive Betätigung des Druckknopfes möglich, wodurch die Klemmzange geöffnet wird. Eine Elektrodenleitung wird eingelegt. Der Druckknopf wird anschließend losgelassen, wodurch die Klemmzange in den Ruhezustand zurückfällt und die Elektrodenleitung geklemmt wird. Hierbei ist es egal, welchen Durchmesser die Elektrodenleitung hat.

Das Design der Klemmung an der Peripherie der Klemmzange ist so gestaltet, dass die Elektrodenleitung in Richtung des Schlitzens gut klemmt. Aufgrund einer leichten Öffnung der Halbschalen im vorderen Bereich sind die Klemmkräfte im rechten Winkel zur Schlitzrichtung geringer. Dies ermöglicht ein selbstständiges Lösen der Elektrodenleitung aus der Fixierung, falls das Schlitzwerkzeug verkantet. Das Schlitzwerkzeug wird in der gleichen Ebene wie der Kathetergriff gehalten (parallel zueinander). Des Weiteren befindet sich der Haltepunkt, an dem Daumen und Zeigefinger das Schlitzwerkzeug halten, auf gleicher Höhe oder noch vor der Klinge. Diese beiden Punkte minimieren ein Verkanten. Mit dem Schlitzwerkzeug wird eine Zieh- und keine Schubbewegung ausgeführt. Somit zentriert es sich selbstständig und verkantet weniger.

Die Fixierung ist dermaßen gestaltet, dass ein Wiedereinführen des Schlitzwerkzeugs in den Katheter problemlos möglich ist.

Weitere mögliche Ausführungen sind in den Figuren gezeigt. Es zeigen
- FIG. 1: das Schlitzwerkzeug gemäß der Ausführung in der Seitenansicht
- FIG. 2: eine Draufsicht auf die Peripherie der Klemmzange des Schlitzwerkzeugs
- FIG. 3A und FIG. 3B: eine schematische Darstellung des Klemmmechanismusses gemäß der Ausführung des Schlitzwerkzeugs

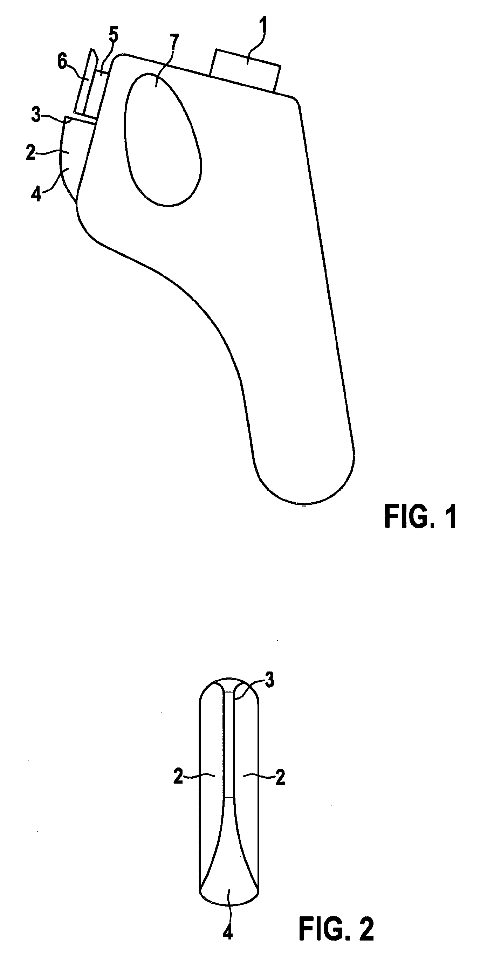

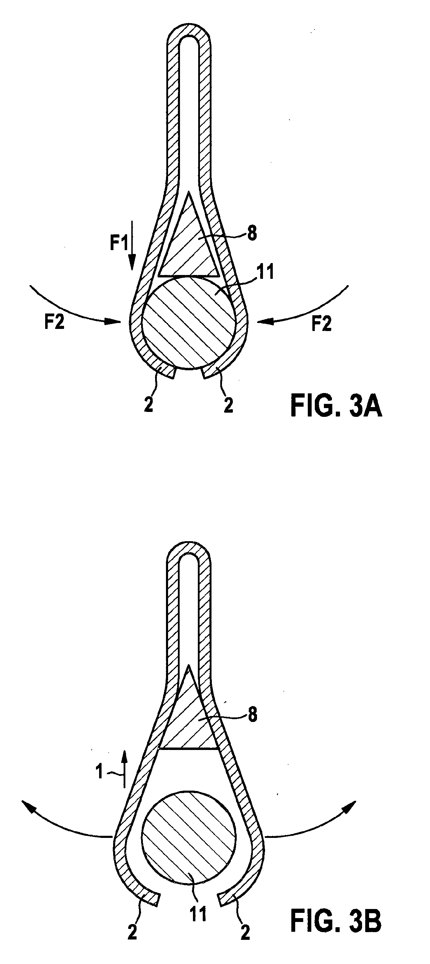

FIG. 1 zeigt das Schlitzwerkzeug der Ausführung in der Seitenansicht. Ein Druckknopf 1 ist über das Spreizelement 8 mit einer Klemmzange 2 verbunden. Wird der Druckknopf 1 gedrückt, führt das zu einer Bewegung des Spreizelementes 8 (siehe Fig. 3A und 3B) Durch diese Bewegung öffnet sich die Klemmzange, das heißt, die sichtbare Peripherie mit den außen liegenden und offenen Klemmzangenschenkeln öffnet sich, indem sie sich voneinander weg entfernen. Das Öffnen der Klemmschenkel wird durch ein Spreizelement 8 bewirkt, das sich zwischen den beiden Schenkeln befindet und sich in die Klemmzange 2 schiebt. Wenn die Elektrodenleitung eingelegt ist, wird der Druckknopf losgelassen. Dadurch geht das Spreizelement in seinen Ruhezustand zurück Gleichzeitig fällt auch die Klemmzange 2 in den Ruhezustand zurück. Der Rückfall in den Ruhezustand wird durch eine oder mehrere Federn bewirkt.

Eine Klinge 5 zum Aufschlitzen des Katheterschaftes entlang seiner Längsachse befindet sich in Schneidrichtung vor der Fixierung. An die Klinge ist zusätzlich eine Elektrodenführung 6 angebracht, welche aus Kunststoff oder Metall gespritzt sein kann. Die Klinge kann entweder zusammen mit der Führung eingesetzt werden oder bei der Herstellung der Führung mit umspritzt werden.

Das Gebilde aus Klinge und Elektrodenführung kann in das Gehäuse eingesetzt oder direkt damit verbunden sein.

Der Haltepunkt 7 (Position von Daumen und Zeigefinger) befindet sich vor oder auf Höhe der Klinge.

Die Peripherie der Klemmschenkel bildet eine Klemmung, die aus zwei runden oder prismaförmigen Halbschalen zur Aufnahme der Elektrodenleitung geformt sind.

FIG. 2 zeigt eine Draufsicht auf die Peripherie der Klemmzange, in die die Elektrodenleitung eingelegt und fixiert werden kann. Erkennbar sind die beiden Schenkel 2 der Klemmzange. Am vorderen, in Richtung Klinge weisenden Ende 3 sind die Schenkel so geformt, dass der Klemmspalt verringert ist. Dort umschließt die Klemmung die Elektrodenleitung. Durch diese Formung ist bei Verkanten des Schlitzwerkzeuges ein selbstständiges Lösen der Elektrodenleitung möglich, was zu der Vermeidung von Elektrodenbeschädigungen führt. Zusätzlich führt dieses Design dazu, dass das Schlitzwerkzeug problemlos wieder in den Katheter eingeführt werden kann. Am hinteren, von der Klinge weg weisenden Ende 4 sind die Schenkel so geformt, dass der Klemmspalt vergrößert ist, wodurch das Einlegen der Elektrodenleitung erleichtert wird.

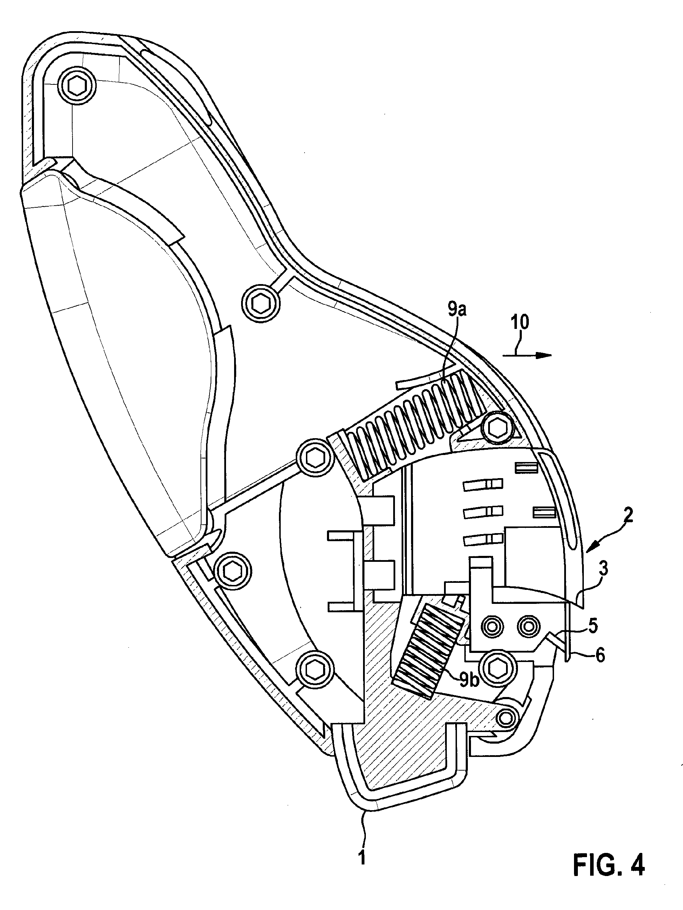

FIG. 3A und FIG. 3B zeigen eine schematische Darstellung des Klemmmechanismusses gemäß der Ausführung. In FIG. 3A ist die Klemmzange 2 im Ruhezustand mit angelegten Schenkeln gezeigt. Es wirken Kräfte F1 und F2 in die durch die Pfeile angezeigten Richtungen, wodurch die Elektrodenleitung 11, die im Schnitt gezeigt ist, entweder gegen das Gehäuse des Schlitzwerkzeuges oder gegen das Spreizelement 8 gedrückt wird. Während die Kräfte F2 durch die Vorspannung der Schenkel 2 bewirkt werden, wird die Kraft F1 durch Federn, wie beispielsweise in FIG. 4 mit 9A und 9B bezeichnet, bewirkt. Beide Federwirkungen werden durch das Betätigen des Druckknopfes 1 aufgehoben.

FIG. 3B zeigt die Klemmzange 2 im aktivierten Zustand, wenn die Fixierung der Elektrodenleitung 11 aufgehoben ist. Durch die Betätigung des Druckknopfes 1 wird die Kraft F1 aufgehoben und das Spreizelement 8 relativ zum Gehäuse und zur Klemmzange 2 bewegt. Das Spreizelement 8 wiederum bewirkt das Aufheben der Kräfte F2, wodurch sich die Schenkel der Klemmzange 2 voneinander weg bewegen und die Fixierung der Elektrodenleitung 11 aufheben.

Vorteile der Gegenstände der Patentanmeldung: Auf der anderen Seite muss sich die Elektrodenleitung senkrecht zur Schlitzrichtung aus der Klemmung lösen können, wenn das Schlitzwerkzeug aus dem Katheter rutscht. Des Weiteren sollte das Design so gewählt werden, dass ein Verkanten des Schlitzwerkzeugs minimiert wird.

## Patentansprüche

1. Schlitzwerkzeug zum Aufschlitzen eines Katheterschaftes, umfassend ein Gehäuse, eine am Gehäuse befestigte Klinge (5), einen am Gehäuse von außen betätigbaren Druckknopf (1) sowie eine im Gehäuse fest gelagerte Klemmzange (2), wobei sich die Klemmzange (2) teilweise innerhalb des Gehäuses befindet, aus zwei Klemmschenkel besteht, welche an ihrer im Gehäuse liegenden Basis miteinander verbunden sind und eine gegenseitige Vorspannung aufweisen, und welche im Ruhezustand an ihrer außerhalb des Gehäuses liegenden Peripherie die Fixierung einer Elektrodenleitung (11) bewirken, und wobei im Gehäuse weiterhin ein Spreizelement (8) verschieblich gelagert ist, und der Druckknopf (1), das Spreizelement (8) und die Klemmzange (2) derart miteinander wirken, dass eine Betätigung des Druckknopfes (1) den Ruhezustand der Klemmzange (2) aufhebt, eine gegenseitige Verschiebung der Klemmzange (2) und des Spreizelements (8) und eine Öffnung der Fixierung in der Peripherie der Klemmzange (2) bewirkt, indem die beiden Schenkel durch das Spreizelement (8) voneinander weg bewegt werden, **dadurch gekennzeichnet, dass** der Druckknopf (1) mit dem Spreizelement (8) verbunden ist, so dass die Betätigung des Druckknopfes (1) eine Verschiebung des Spreizelements (8) in Richtung der im Gehäuse fest gelagerten Basis der Klemmzange (2) bewirkt, so dass die beiden Schenkel auseinandergedrückt werden.

2. Schlitzwerkzeug gemäß Anspruch 1, **dadurch gekennzeichnet, dass** der Druckknopf (1) im Ruhezustand eine erste, aus dem Gehäuse herausragende Position und eine zweite, sich nach der Betätigung im Gehäuse befindliche Position aufweist.

3. Schlitzwerkzeug gemäß Anspruch 2 **dadurch gekennzeichnet, dass** ein oder mehrere Rücksetzelemente eine Rückbewegung des Druckknopfes (1) aus der zweiten in die erste Position bewirken.

4. Schlitzwerkzeug gemäß Anspruch 3, **dadurch gekennzeichnet, dass** das oder die Rücksetzelemente Federn (9) sind, welche zwischen Gehäuse und Druckknopf (1) wirken.

5. Schlitzwerkzeug gemäß einem der Ansprüche 1- 4, **dadurch gekennzeichnet, dass** sich bei Betätigung des Druckknopfes (1) das Spreizelement (8) und die Basis der Klemmzange (2) aufeinander zu verschieben.

6. Schlitzwerkzeug nach einem der Ansprüche 1 - 5, **dadurch gekennzeichnet, dass** das Spreizelement (8) zwischen den beiden Schenkeln der Klemmzange (2) positioniert ist oder bei Betätigung des Druckknopfes (1) die Position zwischen den beiden Schenkel einnimmt.

7. Schlitzwerkzeug nach Anspruch 1, **dadurch gekennzeichnet, dass** die Peripherie der Klemmzange (2) durch die offenen Enden der Klemmzange (2) gebildet sind, welche aus je einer runden oder prismaförmigen Halbschale zur Aufnahme der Elektrodenleitung (11) bestehen, wodurch die Fixierung der Elektrodenleitung (11) während des Aufschlitzens des Katheters bewirkt wird.

8. Schlitzwerkzeug nach Anspruch 7, **dadurch gekennzeichnet, dass** die Halbschalen der Klemmzange (2) im Ruhezustand einen umschlossenen zylinderförmigen Raum bilden, der eine Längsachse aufweist, die so angeordnet ist, dass die in diesem Raum befindliche Elektrodenleitung (11) und damit den Katheter so zur Klinge (5) positioniert sind, dass die Klinge (5) den Katheter in seiner Längsrichtung aufschlitzen kann.

9. Schlitzwerkzeug nach Anspruch 8, **dadurch gekennzeichnet, dass** die Klinge (5) eine Führung aufweist, welche parallel zur Längsachse verläuft.

## Claims

1. A slitting tool for slitting open a catheter shaft, comprising a housing, a blade (5) fastened to the housing, a push button (1), which can be actuated from the outside on the housing, and clamping forceps (2), which are fixedly mounted in the housing, the clamping forceps (2) being partially located inside the housing and composed of two clamping legs, which are connected to each other at the respective bases thereof located in the housing and have a mutual pretension, and which cause the fixation of an electrode lead (11) in the idle state on the peripheries thereof located outside the housing, a spreading element (8) being further displaceably mounted in the housing, and the push button (1), the spreading element (8), and the clamping forceps (2) cooperating with each other in such a way that an actuation of the push button (1) cancels the idle state of the clamping forceps (2) and causes the clamping forceps (2) and the spreading element (8) to be displaced with respect to each other and the fixation in the periphery of the clamping forceps (2) to be opened by moving the two legs away from each other by way of the spreading element (8), **characterized in that** the push button (1) is connected to the spreading element (8) so that the actuation of the push button (1) causes a displacement of the spreading element (8) in the direction of the base of the clamping forces (2) which is fixedly mounted in the housing, so that the two legs are pushed apart from each other.

2. The slitting tool according to claim 1, **characterized in that** the push button (1) has a first position protruding from the housing in the idle state and a second position located in the housing after actuation.

3. The slitting tool according to claim 2, **characterized in that** one or more reset elements cause a return movement of the push button (1) from the second position into the first position.

4. The slitting tool according to claim 3, **characterized in that** the reset element is or the reset elements are springs (9), which act between the housing and the push button (1).

5. A slitting tool according to any one of claims 1 to 4, **characterized in that** spreading element (8) and the base of the clamping forceps (2) are displaced toward each other upon actuation of the push button (1).

6. A slitting tool according to any one of claims 1 to 5, **characterized in that** the spreading element (8) is positioned between the two legs of the clamping forceps (2), or assumes the position between the two legs upon actuation of the push button (1).

7. The slitting tool according to claim 1, **characterized in that** the periphery of the clamping forceps (2) is formed by the open ends of the clamping forceps (2), which are each composed of a round or prism-shaped half shell for accommodation of the electrode lead (11), whereby the fixation of the electrode lead (11) is caused while the catheter is being slit open.

8. The slitting tool according to claim 7, **characterized in that** the half shells of the clamping forceps (2) form an enclosed cylindrical space in the idle state, which has a longitudinal axis that is situated so that the electrode lead (11) located in this space and, thus the catheter, are positioned relative to the blade (5) so that the blade (5) can slit open the catheter in the longitudinal direction thereof.

9. The slitting tool according to claim 8, **characterized in that** the blade (5) has a guide which extends parallel to the longitudinal axis.

## Revendications

1. Outil de rainurage destiné à fendre une tige de cathéter, comprenant un boîtier, une lame (5) fixée au boîtier, un bouton poussoir (1) actionnable de l'extérieur sur le boîtier, ainsi qu'une pince de serrage (2) agencée fixement dans le boîtier, dans lequel la pince de serrage (2) se trouve partiellement à l'intérieur du boîtier, tout en étant constituée de deux jambes de serrage reliées entre elles par leur base située dans le boîtier et exerçant une précontrainte l'une sur l'autre, et provoquant la fixation d'une ligne d'électrode (11), à l'état de repos, sur leur périphérie située à l'extérieur du boîtier, et dans lequel un élément d'espacement (8) est en outre monté de façon coulissante dans le boîtier, et dans lequel le bouton poussoir (1), l'élément d'espacement (8) et la pince de serrage (2) coopèrent de manière à ce qu'un actionnement du bouton poussoir (1) sort la pince de serrage (2) de l'état de repos, provoque un déplacement mutuel de la pince de serrage (2) et de l'élément d'espacement (8) ainsi qu'une ouverture de la fixation à la périphérie de la pince de serrage (2), en éloignant les deux jambes l'une à distance de l'autre à l'aide de l'élément d'espacement (8), **caractérisé en ce que** le bouton poussoir (1) est relié à l'élément d'espacement (8), de sorte que l'actionnement du bouton poussoir (1) provoque un déplacement de l'élément d'espacement (8) dans la direction de la base de la pince de serrage (2) agencée fixement dans le boîtier, de manière à écarter les deux jambes.

2. Outil de rainurage selon la revendication 1, **caractérisé en ce qu'**à l'état de repos, le bouton poussoir (1) présente une première position faisant saillie hors du boîtier et une deuxième position située dans le boîtier après l'actionnement.

3. Outil de rainurage selon la revendication 2, **caractérisé en ce qu'**un ou plusieurs éléments de rappel provoquent un mouvement retour du bouton poussoir (1), de la deuxième vers la première position.

4. Outil de rainurage selon la revendication 3, **caractérisé en ce que** l'élément de rappel ou les éléments de rappel sont des ressorts (9) agissant entre le boîtier et le bouton poussoir (1).

5. Outil de rainurage selon l'une des revendications 1 à 4, **caractérisé en ce que** l'élément d'espacement (8) et la base de la pince de serrage (2) se déplacent l'un vers l'autre lors de l'actionnement du bouton poussoir (1).

6. Outil de rainurage selon l'une des revendications 1 à 5, **caractérisé en ce que** l'élément d'espacement (8) est positionné entre les deux jambes de la pince de serrage (2), ou se met dans la position entre les deux jambes lors de l'actionnement du bouton poussoir (1).

7. Outil de rainurage selon la revendication 1, **caractérisé en ce que** la périphérie de la pince de serrage (2) est formée par les extrémités ouvertes de la pince de serrage (2), lesquelles sont respectivement constituées d'une demi-coque ronde ou prismatique, pour la réception de la ligne d'électrode (11), provoquant ainsi la fixation de la ligne d'électrode (11) pendant le rainurage du cathéter.

8. Outil de rainurage selon la revendication 7, **caractérisé en ce qu'**à l'état de repos, les demi-coques de la pince de serrage (2) forment un espace cylindrique fermé, lequel présente un axe longitudinal agencé de manière à ce que la ligne d'électrode (11) située dans cet espace et donc le cathéter soient agencés de telle façon par rapport à la lame (5), que la lame (5) peut fendre le cathéter dans son sens longitudinal.

9. Outil de rainurage selon la revendication 8, **caractérisé en ce que** la lame (5) comporte un guidage s'étendant parallèlement à l'axe longitudinal.
